# EUROPÄISCHE PATENTSCHRIFT

(11) **EP 1 224 952 B1**
(45) Veröffentlichungstag und Bekanntmachung des Hinweises auf die Patenterteilung: **16.01.2008**
(21) Anmeldenummer: 02090018.9
(22) Anmeldetag: 17.01.2002
(51) Int. Cl.: A61N 1/37

(54) **Stimulationsvorrichtung mit Stimulationserfolgskontrolle**
Stimulation device with capture verification
Stimulation avec verification de capture

(30) Priorität: 17.01.2001 US 262243 P; 08.06.2001 DE 10128982
(43) Veröffentlichungstag der Anmeldung: 24.07.2002
(73) Patentinhaber: BIOTRONIK GmbH & Co. KG, 12359 Berlin (DE)
(72) Erfinder: Greenhut, Saul E., Aurora, Colorado 80015 (US); Nappolz, Tibor, Evergreen, Colorado 80439 (US); Schneider, Gary, Castle Rock, Colorado 80104 (US); Schaldach, Max, Verstorben (DE)
(74) Vertreter: Lindner-Vogt, Karin L.

(56) Entgegenhaltungen:
- EP-A- 0 870 516
- EP-A- 0 941 744
- EP-A- 1 040 849
- US-A- 5 172 690
- US-A- 5 443 485
- US-A- 5 571 144
- US-A- 5 861 012

## Beschreibung

Die Erfindung betrifft eine Stimulationsvorrichtung, welche eine Stimulationseinheit aufweist, die ausgebildet ist, elektrische Stimulationsimpulse zur Stimulation von Körpergewebe abzugeben, sowie eine Auswerteeinheit, die ausgebildet ist, elektrische Signale im Zusammenhang mit der Abgabe eines Stimulationsimpulses aufzunehmen und zur Überprüfung eines Stimulationserfolges auszuwerten.

Die Erfindung betrifft insbesondere einen Herzschrittmacher, wie er üblicherweise verwendet wird, mittels elektrischer Impulse an das Myocard eines Herzens einen Herzrhythmus sicherzustellen, der dem hämodynamischen Bedarf eines Patienten gerecht wird. Solche Herzschrittmacher sind üblicherweise über eine Elektrodenleitung mit Elektroden verbunden, die zur intrakardialen Anordnung und zur elektrischen Stimulation des Herzgewebes (Myocard) durch Abgabe elektrischer Impulse an das Myocard ausgebildet sind. Da solche Herzschrittmacher üblicherweise implantiert werden, ist es von besonderer Bedeutung, dass die mit einem elektrischen Stimulationsimpuls abgegebene Energiemenge gerade so bemessen ist, dass das Herzgewebe sicher stimuliert wird. Ein zu energiereicher Stimulationsimpuls würde die Batterie des Herzschrittmachers zu schnell erschöpfen. Auf der anderen Seite würde ein zu energiearmer Stimulationsimpuls möglicherweise nicht ausreichen, das Herzgewebe im Sinne einer sich ausbreitenden und zur Kontraktion einer entsprechenden Herzkammer führenden Reizung zu erregen.

Vor diesem Hintergrund ist es bekannt, im Zusammenhang mit der Abgabe eines Stimulationsimpulses an das Myocard eine Überprüfung des Stimulationserfolges durch Auswertung im Zusammenhang mit der Abgabe des Stimulationsimpulses auftretender elektrischer Signale durchzuführen, um beispielsweise im Falle eines mangelnden Stimulationserfolges einen energiereicheren Backup Stimulationsimpuls auslösen zu können. Die Stimulationserfolgskontrolle setzt das Erkennen einer erfolgreichen Stimulation voraus, welches auch als Capture Recognition bekannt ist. Es sind zahlreiche Vorrichtungen und Verfahren bekannt, die eine Capture Recognition ermöglichen sollen.

Es wurden bereits eine Vielzahl von Systemen zur automatischen Capture-Detektion entwickelt, die im Zusammenhang mit Herzschrittmachern dazu dienen, die An-/ Abwesenheit einer Depolarisation des Myocards im Anschluss an einen Stimulationsimpuls zu detektieren. Diese Systeme verwenden üblicherweise sehr einfache Methoden wie eine Auswertung der Signalamplitude - d.h. des Maximalbetrags des Signals - um eine einen Stimulationserfolg kennzeichnende Depolarisation des Myocards zu detektieren und auf diese Weise von Signalen zu unterscheiden, die mit einem mangelnden Stimulationserfolg einhergehen. Solche Systeme arbeiten zufriedenstellend, solange die Polarisationsspannung niedrig ist und ein typisches, einen Stimulationserfolg kennzeichnendes Signal auftritt. Eine Verschmelzung von stimulierten und natürlichen Herzreaktionen (Fusionsereignis) kann jedoch dazu führen, dass das auf einen Stimulationsimpuls folgende Signal atypisch ist und von einem einfachen Algorithmus einem mangelnden Stimulationserfolg zugeordnet wird. Außerdem können starke Polarisationsspannungen ein eine erfolgreiche Stimulation kennzeichnendes Signal vortäuschen. Es ist somit ein Problem, das im Myocard evozierte elektrische Signal in Anwesenheit von solchen Signal-Artefakten sicher zu detektieren, die beibeispielsweise auf Polarisationseffekte im Bereich der Grenzfläche zwischen Elektrode und Körperfluid bzw. Myocard zurückgehen.

Verschiedene Lösungen für das letztgenannte Problem werden beispielsweise in den US-Patenten 5,417,718, 5,697,957, 5,861,013, 5,873,898 und 5,941,903 sowie der Europäischen Patentanmeldung EP-A 0 826 392 vorgeschlagen.

Es ist auch bekannt, für einen positiven Stimulationserfolg charakteristische, evozierte Signale anhand typischer Signalformen zu detektieren.

Aus EP 1 040 849 ist es schließlich bekannt, in kurzer Zeit paarweise aufeinanderfolgende Stimulationsimpulse abzugeben, von denen jeweils der erste oder der zweite Stimulationsimpuls einen Stimulationserfolg nach sich zieht. Die erste der beiden Stimulationsimpulse besitzt eine variable Impulsstärke, während der zweite Stimulationsimpuls eine mit Sicherheit überschwellige Stimulationsstärke aufweist. Wenn der erste Stimulationsimpuls zu einem Stimulationserfolg führt, kann der zweite, überschwellige Stimulationsimpuls aufgrund der Tatsache, dass das Myokard durch den ersten Stimulationsimpuls refraktär ist, keine Stimulationserfolg nach sich ziehen. Ist hingegen der erste Stimulationsimpuls unterschwellig, führt erst der zweite Stimulationsimpuls zu einem Stimulationserfolg. Gemäß EP 1 040 849 werden die unmittelbar auf die Abgabe der Stimulationsimpulse jeweils auftretenden Signalverläufe aufgenommen und ein Differenzsignal zwischen diesen beiden Signalverläufen gebildet, dessen Amplitude im wesentlichen davon abhängt, ob bereits der erste Stimulationsimpuls zu einem Stimulationserfolg geführt hat (also überschwellig war) oder erst der zweite Stimulationsimpuls (falls der erste Stimulationsimpuls unterschwellig war). Aus EP 1 040 849 ist somit bekannt, eine Stimulationserfolgskontrolle durch Vergleichen eines Antwortsignals im Falle erfolgreicher Stimulation mit einem Antwortsignal im Falle erfolgloser Stimulation durchzuführen.

Aus Dokument US 5 172 690 ist eine Stimulationsvorrichtung bekannt, die das nach Abgabe eines Stimulationsimpulses auftretende Signal aufnimmt und über eine Zeit von 40 ms nach einer Blanking-Periode integriert. Wenn das integrierte Signal einen Schwellwert nicht überschreitet, meldet die Vorrichtung einen Fall mangelnden Stimulationserfolges.

Erfindungsgemäß wird dieses Integral über die Zeit bestimmt, in der das nach der Blanking-Periode gemessene Signal oberhalb der Signalamplitude während der Blanking-Periode verläuft.

Leider ist das Erkennen des Stimulationserfolges in allen Fällen mit statistischen Unsicherheiten behaftet, so das nach wie vor der Wunsch besteht, eine Capture Recognition mit höherer Spezifität und Sensitivität durchzuführen.

Die Erfindung beruht insbesondere auf der Erkenntnis, dass ein auf einen Stimulationsimpuls folgendes Signal zwei separate Signalelemente aufweist, nämlich zum einen ein erstes Signalelement, das auf die Polarisation des Myocard zurückgeht und zum anderen ein zweites Signalelement, welches auf einer Polarisation im Bereich des an die Stimulationselektrode angrenzenden Gewebes bzw. Körperfluids beruht. Diese Polarisation im Bereich der Elektrodengrenzfläche stellt verschwendete Energie dar und führt zu einem Signal, welches mit der positiven Detektion eines evozierten Signals im Anschluss an eine Stimulation interferiert. Zwar sind moderne Elektrodenleitungen, beispielsweise fraktale Elektrodenleitungen so gestaltet, dass die Polarisationspotentiale möglichst reduziert werden. Dies wird typischerweise über eine Vergrößerung der Elektrodenoberfläche durch Mikrostrukturierung erzielt, wodurch kapazitive Effekte reduziert werden. Obwohl jedoch mit solchen Elektroden die Polarisation vermindert wird, wird sie nicht gänzlich eliminiert. Außerdem variieren die Elektroden innerhalb und zwischen diversen Modellreihen hinsichtlich ihrer Effektivität im Reduzieren von Polarisationspotentialen. Und selbst die besten Elektroden zeigen bei Stimulationsamplituden von mehr als 2 Volt messbare, auf die Polarisation zurückzuführende Signalartefakte selbst noch 20 Millisekunden nach einem Stimulationsimpuls. Die Amplitude dieses Artefakts vergrößert die Spannung und verbreitert den Stimulationsimpuls.

Bei der erfindungsgemäßen Stimulationsvorrichtung ist es erstmalig vorgesehen, für die Polarisation charakteristische Signalartefakte als einen mangelnden Stimulationserfolg kennzeichnendes Signalmerkmal zu detektieren.

Dies beruht auf der Erkenntnis, das derartige, auf die Polarisation zurückzuführende Signalartefakte regelmäßig eine ähnliche Morphologie aufweisen. Diese Erkenntnisse wurden durch Versuche gewonnen, bei denen Elektrodenleitungen in einer Salzlösung auf die Signalformen hin untersucht wurden, die sich im Anschluss an die Abgabe eines Impulses an die Salzlösung ergeben.

Auf der anderen Seite wurde beobachtet, dass im Menschen aufgenommene Elektrogramme beinahe jede Form annehmen können. Solche Variationen von Elektrogramm - Morphologien können sich sowohl bei verschiedenen Messungen an einem Patient als auch bei Messung an verschiedenen Patienten ergeben. Unterschiede zwischen den Messungen an einem Patienten ergeben sich typischerweise aus Fusionsereignissen, Atmungseinflüssen einer anodalen Stimulation oder aus anderen, nicht identifizierten Gründen. Unterschiede zwischen Messungen an unterschiedlichen Patienten ergeben sich vermutlich aus Unterschieden in der kardialen Pathologie, der Elektrodenleitungsposition oder der Elektrodenpolarisation.

Auf Basis der Polarisationssignalform wurden charakteristische Merkmale, Signaturen des Polarisationspotentials in Form einer Vielzahl von Signalmerkmalen und Schwellwerten extrahiert. Bevorzugte Ausführungsvarianten der Erfindung stellen auf diese Signalmerkmale und Schwellwerte ab.

Zu den detektierten Merkmalen zählen ein erstes Integral INGR1 des nach Ablauf einer Blanking Periode nach Abgabe eines Stimulationsimpulses gemessen Signals über die Zeit bis zu dem Zeitpunkt, indem das gemessene Signal den während der Blanking Periode herrschenden Signalwert kreuzt. Dieses erste Signal ist in der Regel positiv, da das im Anschluss an die Blanking Periode gemessene Signal üblicherweise über den Signalwert während der Blanking Periode liegt. Entsprechend ist die Auswerteeinheit vorzugsweise dazu ausgebildet, das erste Integral INGR1 zu bestimmen.

Ein weiteres vorzugsweise aufgenommenes und ermitteltes Signalmerkmal ist ein zweites Integral INGR2 des gemessenen Signals, welches über einen Zeitraum gebildet wird, welcher mit dem Zeitpunkt beginnt, für den das erste Integral endet und welcher mit dem Ende eines vorgegebenen Zeitfensters endet, wobei das vorgegebene Zeitfenster mit dem Ablauf der Blanking Periode beginnt. Entsprechend ist die Auswerteeinheit vorzugsweise dazu ausgebildet, auch das zweite Integral INGR2 zu bestimmen.

Ein weiteres Signalmerkmal, welches vorzugsweise durch eine entsprechend ausgebildete Auswertereinheit aufgenommen wird, ist die Anzahl CNT1 der Tastwerte des aufgenommenen Signals, welche in die Zeit fallen, über die das erste Integral gebildet wird. Die Stimulationsvorrichtung ist in diesem Falle dazu ausgebildet, das aufgenommene Signal zeitdiskret abzutasten, so dass das aufgenommene Signal in Form einer Vielzahl zeitdiskreter Tastwerte (samples) vorliegt. Zum Zählen der Tastwerte innerhalb eines Zeitraumes weist die Auswerteeinheit vorzugsweise einen Zähler auf.

Ein weiteres zusätzlich oder auch alternativ aufgenommenes Signalmerkmal besteht in einem Indikator-Flag CROSS, dessen Wert davon abhängt, ob das gemessene Signal während der Zeitdauer für die Bestimmung des zweiten Integrals die während der Blanking Periode herrschende Signalamplitude kreuzt. Wenn der Signalwert während der Blanking Amplitude für das anschließend gemessene Signal den Referenz- oder Nullwert bildet, kennzeichnet das Indikator-Flag CROSS=1 das Vorliegen eines Nulldurchgangs für das gemessene Signal während der Zeit, über die das zweite Integral gebildet wird. Liegt kein Nulldurchgang vor ist das Indikator-Flag CROSS=0. Die Auswerteeinheit ist vorzugsweise zum Bestimmen und Speichern des binären Wertes des Indikator-Flags CROSS ausgebildet.

Ein weiteres Signalmerkmal ist der maximale positive Tastwert des nach Abschluss der Blanking Periode aufgenommenen, gemessenen Signals unter Nichtberücksichtigung der ersten x Tastwerte nach Ablauf der Blanking Periode. Es wird somit vorzugsweise der x+1. Tastwert nach Ablauf der Blanking Periode als maximaler positiver Tastwert MAX_POS aufgenommen. Der Tastwert wird - wie das gesamte nach Ablauf der Blanking Periode gemessene Signal - relativ zu der während der Blanking Periode herrschenden Signalamplitude bestimmt. Außerdem werden vorzugsweise alle im Bezug auf die Signalamplitude während der Blanking Periode negativen Tastwerte als NEG_AMP gespeichert. Die Auswerteeinheit ist entsprechend vorzugsweise zum Bestimmen des maximalen positiven Tastwertes MAX_POS sowie der negativen Tastwerte NEG_AMP und zum Speichern dieser Werte ausgebildet.
Zur weiteren Analyse der im vorbeschriebenen Sinne bestimmten Signalmerkmale weist die Auswerteeinheit vorzugsweise verschiedene Schwellwerteinheiten auf, die im wesentlichen einen Speicher für den jeweils vorgegebenen Wert des entsprechenden Schwellwertes umfassen, sowie eine Vergleichseinheit, die ein Signal ausgibt, welches davon abhängt, ob der vorgegebene Schwellwert über-oder unterschritten wird. Folgende Schwellwerte bzw. Parameter sind vorzugsweise vorgesehen:
- w: Breite des Zeitfensters in Millisekunden oder Anzahl von Tastwerten
- w1: Grenzwert für einen ersten positiven Signalabschnitt, der durch die Anzahl der Tastwerte gegeben ist, über die das erste Integral gebildet wird (CNT1)
- zn: Negativer Grenzwert für die auf die Signalamplitude während der Blanking Periode bezogenen Tastwerte
- zp: Grenzwert für den maximalen positiven Tastwert MAX_POS falls der positive Signalabschnitt breiter ist als durch w1 vorgegeben
- a1: Ein Grenzwert für eine Fläche AREA welche aus dem ersten und dem zweiten Integral (INGR1, INGR2) gebildet wird als Kennzeichen für ein Nicht-Capture
- a 2: Grenzwert für die Fläche AREA, oberhalb dessen ein Capture vorliegt
- x: Die bereits vorgenannte Anzahl von Tastwerten nach Ende der Blanking Periode, die für die Bestimmung von MAX_POS ignoriert werden

Die Auswerteeinheit ist weiterhin vorzugsweise ausgebildet, eine Ermittlung einer nicht erfolgreichen Stimulation (Non-Capture) basierend auf den zuvor eingeführten Signalmerkmalen und Grenzwerten nach folgendem Algorithmus durchzuführen:

```
 If NEG_AMP < zn Then Capture

 If CNT1 > w1 Then AREA = INGR1+INGR2 Else AREA = INGR2

 If AREA < a1 Then Non-Capture

 Elseif CNT1 > w1 Then (If MAX_POS<zp Then Non-Capture Else Capture)

 Elseif AREA > a2 Then Capture

 Elseif CROSS = 1 Then Capture

 Else Non-Capture
```

Die Auswerteeinheit ist somit vorzugsweise dazu ausgebildet die folgenden Schwellwertvergleiche durchzuführen und entsprechende Ergebnisse zu liefern:

Wenn NEG_AMP größer ist als zn gibt die Auswerteeinheit ein eine erfolgreiche Stimulation kennzeichnendes Capture-Signal aus.
Wenn die Anzahl der Tastwerte CNT1 größer ist als w1, dann wird der Flächenwert AREA als Summe aus dem ersten Integral INGR1 und dem zweiten Integral INGR2 gebildet, ansonsten ist der Flächenwert AREA gleich dem zweiten Integral INGR2. Anschließend wird die Fläche AREA mit dem Schwellwert a1 verglichen und wenn AREA kleiner ist als a1 ein eine erfolglose Stimulation kennzeichnendes Non-Capture Signal ausgegeben. Falls diese Bedingung nicht erfüllt ist prüft die Auswerteeinheit, ob die Anzahl CNT1 größer ist als der Grenzwert w1 und führt, falls dies der Fall ist, einen Vergleich des maximalen positiven Tastwertes MAX_POS mit dem Grenzwert zp durch. Ist MAX_POS kleiner ist als zp wird ein Non-Capture Signal ausgegeben, ansonsten ein Capture Signal. Falls keine der beiden Bedingungen AREA kleiner ist als a1 und CNT1 größer ist als w1 gegeben sind, vergleicht die Auswerteeinheit den Flächenwert AREA mit dem Grenzwert a2 und gibt für den Fall, dass AREA größer als a2 ist, ein Capture Signal aus. Trifft auch diese Bedingung nicht zu, prüft die Auswerteeinheit, ob das Indikator-Flag CROSS auf 1 gesetzt ist, ob also das gemessene Signal im Bereich des zweiten Integrals einen Nulldurchgang aufweist. In diesem Fall wird ein Capture Signal ausgegeben. Trifft auch diese Bedingung nicht zu, gibt die Auswerteeinheit ein Nicht-Capture Signal aus.

Darüber hinaus ist die Auswerteeinheit vorzugsweise dazu ausgebildet, schon beim Aufnehmen der Tastwerte für das gemessene Signal ständig ein Vergleich der Tastwerte mit dem Schwellwert für die negative Signalamplitude zn durchzuführen. Falls ein negativer Tastwert NEG_AMP kleiner ist als zn (oder mit anderen Worten einen größeren negativen Betrag hat als zn) wird das gemessene Signal sofort als einen Stimulationserfolg kennzeichnendes Signal klassifiziert und dementsprechend ein Capture Signal ausgegeben. Diese Überprüfung führt die Auswerteeinheit vorzugsweise Tastwert für Tastwert parallel zu dem zuvor beschriebenen Algorithmus durch, und bricht diesen Algorithmus sofort ab, falls ein Tastwert NEG_AMP kleiner ist als zn. Der zuvor beschriebene Algorithmus wird nur dann bis zum Ende fortgeführt, wenn die Bedingung NEG_AMP < zn während des Aufnehmens der Tastwerte nicht erfüllt wird. Dies spart in vorteilhafter Weise Rechenkapazität und Energie.

Soweit eine kurze Beschreibung der bevorzugten Ausgestaltung der Erfindung.

Diese soll nun anhand eines Ausführungsbeispiels und mit Hilfe der Figuren näher erläutert werden.

Von den Figuren zeigen
- Fig.1:: ein schematisches Blockschaltbild eines erfindungsgemäßen Herzschrittmachers
- Fig.2:: eine Darstellung einer typischerweise nach Abgabe eines Stimulationsimpulses aufgenommenen Signals über die Zeit mit den typischen Signalmerkmalen für ein Polarisations-Artefakt samt Darstellung der vorzugsweise aufgenommen und vorgegebenen Signalmerkmale und Schwellwerte;
- Fig.3:: eine schematische Darstellung einer typischen Signalform eines nach einem Stimulationsimpuls aufgenommenen Signals im Falle einer erfolgreichen Stimulation mit den dazugehörigen aufgenommenen Signalmerkmalen und Schwellwerten;
- Fig.4:: eine Signalform des aufgenommenen Signals nach erfolgreicher Stimulation mit anfänglicher positiver Amplitude

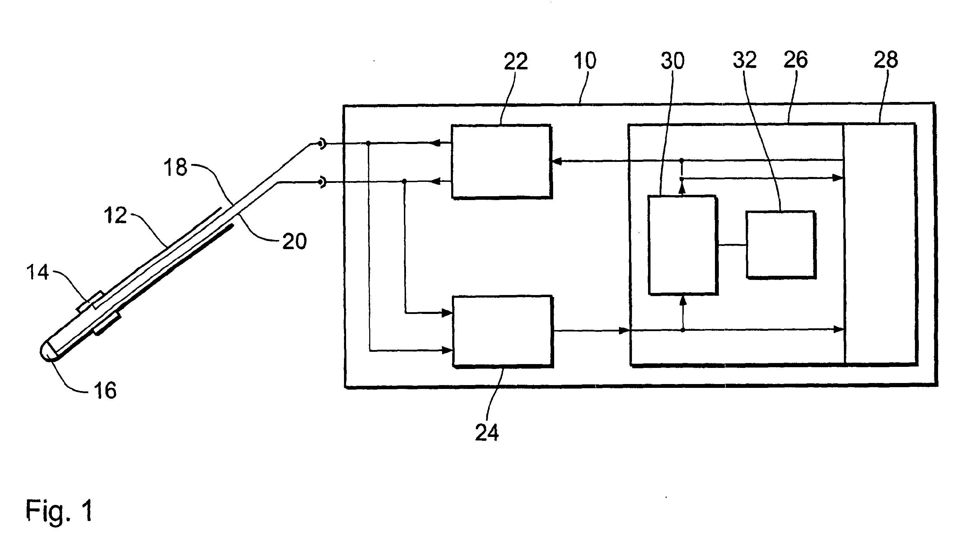

Figur 1 zeigt einen Herzschrittmacher 10 mit daran angeschlossener Elektrodenleitung 12. Die Elektrodenleitung 12 ist ausgebildet um beispielsweise in einen Ventrikel eines menschlichen Herzens eingeführt zu werden. Die Elektrodenleitung 12 ist als bipolare Elektrodenleitung ausgeführt und besitzt daher eine Ringelektrode 14 und eine Spitzenelektrode 16. Die Ringelektrode 14 und die Spitzenelektrode 16 sind über zwei elektrische Leitungen 18 bzw. 20 mit dem Herzschrittmacher 10 und insbesondere einer Stimulationseinheit 22 sowie einer Detektionseinheit 24 des Herzschrittmachers 10 verbunden.

Die Stimulationseinheit 22 ist dazu ausgebildet über die Elektroden 14 und 16 bipolare Stimulationsimpulse im Ventrikel eines Herzens abzugeben. Alternativ kann die Stimulationseinheit 22 auch nur über eine elektrische Leitung wie die elektrische Leitung 20 mit der Spitzenelektrode 16 verbunden sein, um Stimulationsimpulse im unipolaren Modus zwischen einem Gehäuse des Herzschrittmachers 10 und der Spitzenelektrode 16 abzugeben.

Die Detektionseinheit 24 ist mit den Elektroden 14 und 16 verbunden um elektrische Signale des Herzens aufzunehmen. Auch dies kann wie in Figur 1 dargestellt im bipolaren Modus geschehen, oder alternativ in einem unipolaren Modus über die Spitzenelektrode 16 oder die Ringelektrode 14. Die Detektionseinheit 24 dient der Signalaufnahme und der Anpassung der Messsignale wie möglicherweise einer Impedanzwandlung. Außerdem steuert die Detektionseinheit 24 mögliche Ausblendzeiten (Blanking Perioden) oder Refraktärzeiten des Herzschrittmachers 10, in denen gemessene Signale nicht ausgewertet oder gar nicht erst aufgenommen werden. Die Aufnahme der Signale erfolgt somit in zeitlicher Zuordnung zu möglicherweise über die Stimulationseinheit 22 abgegebenen Stimulationsimpulsen. Die aufgenommenen Signale stellen im Wesentlichen ein intrakardial gewonnenes Elektrokardiogramm dar, welches schrittmacher-intern zur weiteren Auswertung an eine Steuereinheit 26 weitergeleitet wird. Die Steuereinheit 26 umfasst eine Stimulationssteuereinheit 28, die in an sich bekannter Weise der Ansteuerung der Stimulationseinheit 22 in Abhängigkeit der intrakardial gewonnenen Signale und möglicher weiterer Signale dient, wie beispielsweise physiologischen Steuersignalen zur bedarfsgerechten, ratenadaptiven Stimulation des Herzens. Die Stimulationssteuereinheit 28 kann auch mit einem Sensor im Vorhof eines Herzens verbunden sein, um eine atriumssynchrone Stimulation des Ventrikels in bekannter Weise durchzuführen. Außerdem bietet die Stimulationssteuereinheit 28 die an sich bekannte Funktion, die Abgabe von ventrikulären Stimulationsimpulsen zu unterdrücken, wenn innerhalb eines vorgegebenen Zeitfensters über die Detektionseinheit 24 eine natürliche Ventrikelaktion detektiert wird. Um die Stimulationseinheit 22 entsprechend ansteuern zu können, ist die Stimulationssteuereinheit 28 mit der Stimulationseinheit 22 verbunden.

Soweit der Herzschrittmacher 10 bis hierher beschrieben wurde, kann er in jeder aus dem Stand der Technik bekannten Ausführungsform realisiert sein.

Wesentliches Merkmal des Herzschrittmachers 10 ist eine Auswerteeinheit 30, der eingangsseitig ein intrakardial aufgenommenes, beispielsweise von der Detektionseinheit 24 stammendes Signal zugeführt wird. Dieses Signal kann grundsätzlich auf jede bekannte Art gewonnen werden, die zum Aufnehmen ventrikulärer evozierter Reizantworten (VER) geeignet ist. Die Auswerteeinheit 30 ist ausgebildet, dass eingangsseitig anliegende Signal von einem Zeitpunkt nach Ende einer Blanking Periode oder Ausblendzeit auszuwerten, die ihrerseits mit der Abgabe eines Stimulationsimpulses durch die Stimulationseinheit 22 beginnt. Die Auswertung des Signals nach Ablauf der Blanking Periode erfolgt mit Bezug auf einen während der Blanking Periode herrschenden Signalwert. Dieser stellt für die weitere Auswertung des Signals praktisch den Nullpunkt dar. Die Auswertung des Signals erfolgt auf die einleitend beschriebene Weise durch Extraktion der Signalmerkmale INGR1, INGR2, CNT1, CROSS, MAX_POS und NEG_AMP. Weiterhin ist die Auswerteeinheit 30 zur Durchführung des eingangsbeschriebenen Algorithmussees ausgebildet. Sie ist dazu mit einem Speicher 32 für alle zu speichernden und vorzugebenden Werte, insbesondere die Werte w, w1, zn, zp, a1, a2, x und AREA verbunden.

Die Auswerteeinheit 30 ist insbesondere dazu ausgebildet, alle Tastwerte NEG_AMP ständig mit dem Grenzwert zn zu vergleichen und bei positiven Vergleichsergebnis ein eine erfolgreiche Stimulation kennzeichnendes Capture-Signal an die Stimulationssteuereinheit 28 abzugeben.

Wesentlich ist jedoch, dass die Auswerteeinheit 30 unter den Eingangs beschriebenen Bedingungen auch ein eine erfolglose Stimulation kennzeichnendes Non-Capture Signal ausgibt. In Figur 1 ist eine Variante dargestellt, in der dieses Non-Capture Signal direkt auf eine zur Stimulationseinheit 22 führende Steuerleitung gegeben wird, um unmittelbar einen Back-up Stimulationsimpuls auszugeben, falls die Auswerteeinheit 30 einen Stimulationsmisserfolg detektiert hat. Dieses Non-Capture Signal wird gleichzeitig an die Stimulationssteuereinheit 28 abgegeben. Es sind Ausführungsvarianten denkbar, in denen die Auswerteeinheit 30 nur im Falle eines Stimulationsmisserfolges ein Non-Capture Signal direkt an die Stimulationseinheit 22 abgibt. In einer anderen Ausführungsvariante können sowohl das Capture Signal als auch das Non-Capture Signal oder nur eines der beiden Signale ausschließlich direkt an die Stimulationssteuereinheit 28 abgegeben werden.

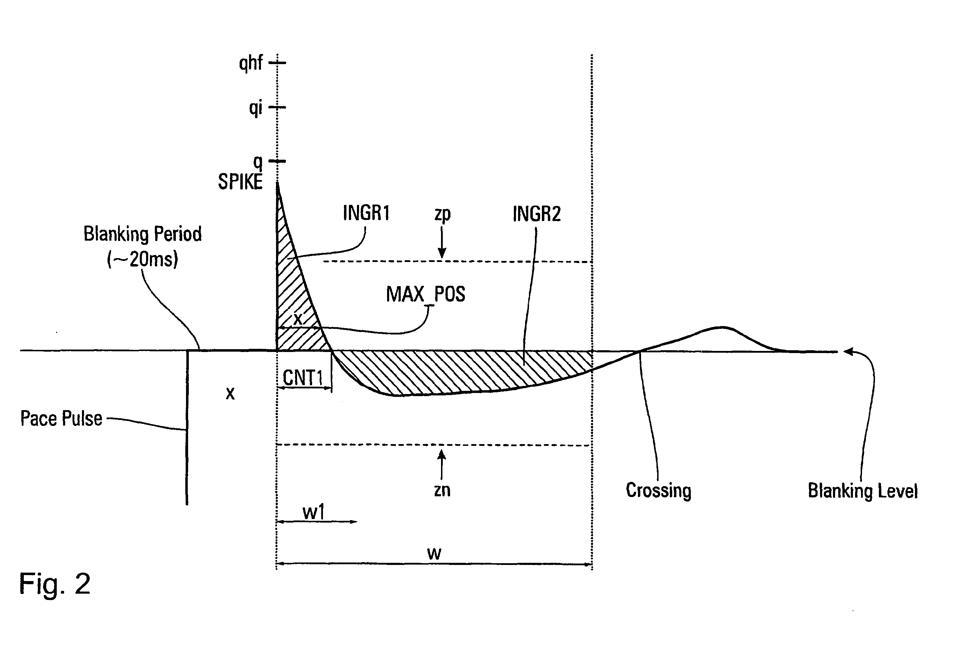

Figur 2 zeigt eine typische, auf die beschriebene Polarisation zurückgehende Signalform, das heißt einen Polarisationsartefakt. In Figur 2 sind all die vorgenannten Signalmerkmale eingetragen, zu deren Extraktion die Auswerteeinheit 30 ausgebildet ist, sowie alle vorgegebenen Grenz- und sonstigen Werte, die in dem Speicher 32 gespeichert sind. Als "Blanking level" ist außerdem die während der Blanking Periode herrschende Signalamplitude als Nulllinie eingetragen. Die Blanking Periode dauert im dargestellten Fall 20 Millisekunden nach Abgabe eines Stimulationsimpulses. Der Zeitpunkt der Abgabe des Stimulationsimpulses ist ebenfalls dargestellt. Wie Figur 2 zu entnehmen ist, beginnt das nach Ende der Blanking Periode aufgenommene Signal mit einem positiven Signalspitzenwert, fällt dann ab, um nach einer bestimmten Anzahl von Tastwerten (CNT1) unter das während der Blanking Periode herrschende Signal zu fallen. Das dann negative Signal steigt nach einiger Zeit wieder an und kreuzt das während der Blanking Periode herrschende Signalniveau. Wenn dieser Kreuzpunkt innerhalb des durch w vorgegebenen Zeitfensters liegt, wird das Indikator-Flag auf 1 gesetzt. Liegt der Kreuzungspunkt - wie in dem in Figur 2 dargestellten Fall - au-βerhalb des durch w vorgegebenen Zeitfensters, bleibt das Indikator-Flag CROSS null. Dargestellt ist auch das positive erste Intergral INGR1, welches das Integral des Messsignals über die Zeit beginnend mit Ende der Blanking Periode bis zum ersten negativen Nulldurchgang des Signals ist, sowie das zweite Integral INGR2, welches das verbleibende Flächenintegral des Messignals in dem durch w vorgegebenen Zeitfenster in Bezug auf die während der Blanking Periode herrschende Signalamplitude darstellt.

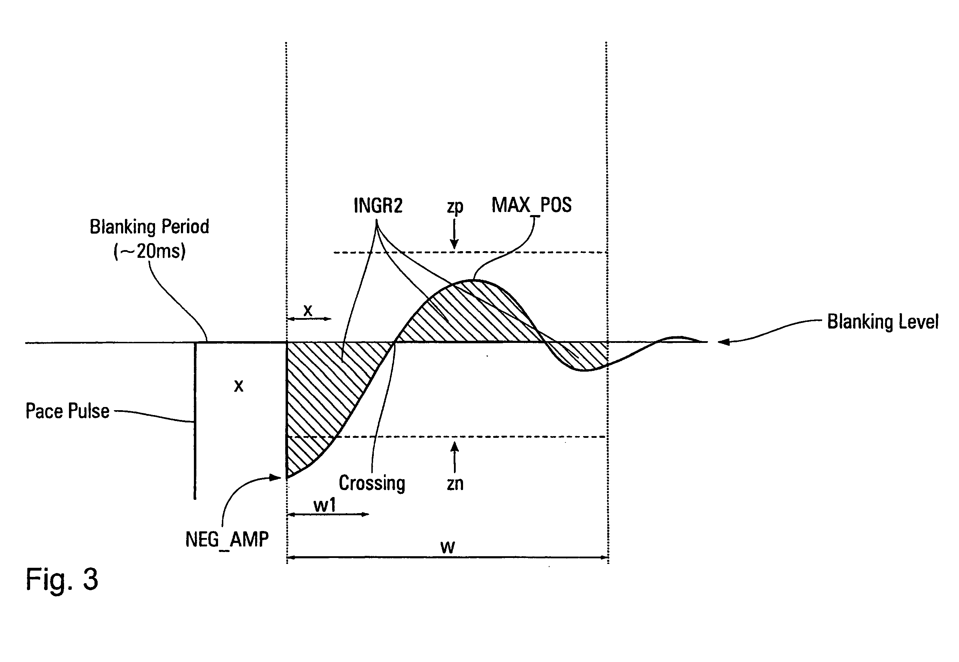

Figur 3 zeigt den typischen Verlauf einer evozierten Reizantwort und aller zuvor beschriebenen Parameter. Die extrahierten Signalmerkmale sind wie zuvor in Großbuchstaben dargestellt, während die Grenzwerte und sonstige vorzugebenen Parameter mit kleinen Buchstaben bezeichnet sind. Das in Figur 3 dargestellte, einen positiven Stimulationserfolg kennzeichnende Signal beginnt mit einer negativen Amplitude, deren negativer Betrag größer ist, als der Grenzwert zn. Bereits mit dem ersten Tastwert steht somit fest, dass das Signal einem positiven Stimulationserfolg zuzuordnen ist, so dass die Auswerteeinheit 30 den zuvor beschriebenen Algorithmus bereits mit dem ersten Tastwert abbrechen und ein Capture Signal ausgeben kann.

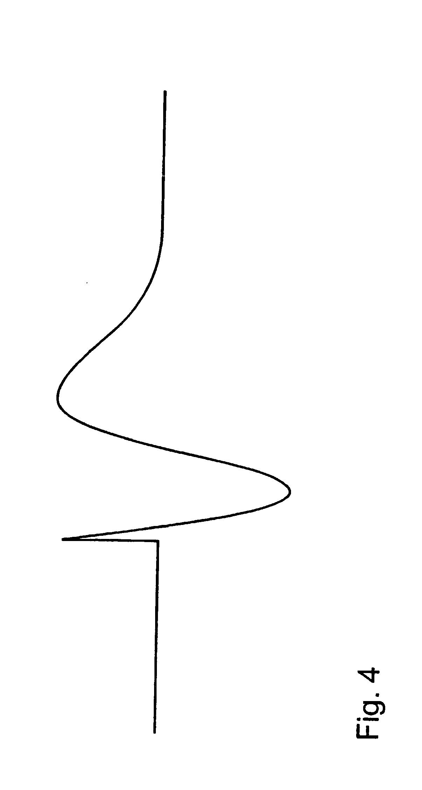

Wie in Figur 4 dargestellt kann das im Falle eines Stimulationserfolges gewonnene Signal auch mit einem positiven Signalspitzenwert beginnen. Auch in diesem Falle erlaubt die Auswerteeinheit 30 eine zuverlässige Erkennung eines Stimulationsmisserfolges und gibt im Falle der in Figur 4 dargestellten Signalform kein Non-Capture sondern ein Capture Signal aus.

Über die in dem Ausführungsbeispiel dargestellte Variante hinaus erstreckt sich die Erfindung auch auf alle anderen, für den Fachmann naheliegenden Varianten, wie beispielsweise die bereits angedeutete Variante, bei der die Auswerteeinheit 30 die Stimulationseinheit 22 im Non-Capture Fall direkt ansteuert, um einen Back-up Stimulationsimpuls mit höherer Energie als dem vorangegangenen Stimulationsimpuls auszulösen.

## Patentansprüche

1. Stimulationsvorrichtung, welche eine Stimulationseinheit aufweist, die ausgebildet ist elektrische Stimulationsimpulse zur Stimulation von Körpergewebe abzugeben, sowie eine Auswerteeinheit, die ausgebildet ist, intrakardiale elektrische Signale im Zusammenhang mit der Abgabe eines Stimulationsimpulses aufzunehmen und zur Überprüfung eines Stimulationserfolges auszuwerten,
wobei die Auswerteinheit ausgebildet ist in dem intrakardial aufgenommenen Signal solche Signalmerkmale zu detektieren, die für einen Fall mangelnden Stimulationserfolges spezifisch sind und ein entsprechendes Ausgangssignal abzugeben, wobei die Auswerteeinheit ausgebildet ist, ein aufgenommenes elektrisches Signal zeitlich einem Stimulationsimpuls zuzuordnen und ein Merkmal eines Polarisationsartefaktes als Signalmerkmal in dem aufgenommenen Signal zu detektieren- und
das nach Ablauf einer Blanking-Periode nach Abgabe eines Stimulationsimpulses gemessene Signal auszuwerten und zum Detektieren eines Merkmals eines Polarisationsartefaktes ein erstes Integral (INGR1) des gemessenen Signals über die Zeit zu bestimmen, in der das nach der Blanking-Periode gemessene Signal oberhalb der Signalamplitude während der Blanking-Periode verläuft.

2. Stimulationsvorrichtung nach Anspruch 1, **dadurch gekennzeichnet, dass** die Auswerteeinheit ausgebildet ist, ein zweites Integral (INGR2) des gemessenen Signals über eine Zeitdauer zu bestimmen, welche mit dem Zeitpunkt beginnt, für den das erste Integral endet, und welche mit dem Ende eines vorgegebenen Zeitfensters endet, welches mit dem Ende der Blanking-Periode beginnt.

3. Stimulationsvorrichtung nach Anspruch 1, bei der das nach der Abgabe eines Stimulationsimpulses aufgenommene Signal in Form von zeitlich diskreten Tastwerten aufgenommen wird, **dadurch gekennzeichnet, dass** die Auswerteeinheit einen Zähler umfasst, der ausgebildet ist, die Anzahl (CNT1) der Tastwerte des aufgenommenen Signals zu bestimmen, die in die Zeit fallen, über die das erste Integral gebildet wird.

4. Stimulationsvorrichtung nach Anspruch 2, **dadurch gekennzeichnet, dass** die Auswerteeinheit ausgebildet ist, ein Indikator-Flag (CROSS) zubilden, dessen Wert davon abhängt, ob das gemessene Signal während der Zeitdauer für die Bestimmung des zweiten Integrals die während der Blanking-Periode herrschende Signalamplitude kreuzt.

5. Stimulationsvorrichtung gemäß den Ansprüchen 1 bis 4, **dadurch gekennzeichnet, dass** die Auswerteeinheit ausgebildet ist, den folgenden Algorithmus auf ein Eingangssignal anzuwenden:
If NEG_AMP < zn Then Capture
If CNT1 > w1 Then AREA = INGR1+INGR2 Else AREA = INGR2
If AREA < a1 Then Non-Capture
Elseif CNT1 > w1 Then (If MAX_POS<zp Then Non-Capture Else Capture)
Elseif AREA > a2 Then Capture
Elseif CROSS = 1 Then Capture
Else Non-Capture
mit
NEG_AMP negative Tastwerte des gemessenen Signals
INGR1 erstes Integral des gemessenen Signals über die Zeit, in der das nach der Blanking-Periode gemessene Signal oberhalb der Signalamplitude während der Blanking-Periode verläuft.
INGR2 zweites Integral des gemessenen Signals über eine Zeitdauer zu bestimmen, welche mit dem Zeitpunkt beginnt, für den das erste Integral endet, und welche mit dem Ende eines vorgegebenen Zeitfensters endet, welches mit dem Ende der Blanking-Periode beginnt
CNT1 Zahl von Tastwerten, über die das erste Integral gebildet wird
AREA Fläche, welche aus dem ersten und dem zweiten Integral (INGR1, INGR2) gebildet wird als Kennzeichen für ein Nicht-Capture
MAX_POS maximaler positiver Tastwert
CROSS Indikator-Flag, dessen Wert davon abhängt, ob das gemessene Signal während der Zeitdauer für die Bestimmung des zweiten Integrals die während der Blanking-Periode herrschende Signalamplitude kreuzt
w1 Grenzwert für einen ersten positiven Signalabschnitt, der durch die Anzahl der Tastwerte gegeben ist, über die das erste Integral gebildet wird (CNT1)
zn Negativer Grenzwert für die auf die Signalamplitude während der in der Blanking Periode bezogenen Tastwerte
zp Grenzwert für den maximalen positiven Tastwert MAX_POS falls der positive Signalabschnitt breiter ist als durch w1 vorgegeben
a1 Grenzwert für eine Fläche AREA welche aus dem ersten und dem zweiten Integral (INGR1, INGR2) gebildet wird als Kennzeichen für ein Nicht-Capture
a 2 Grenzwert für die Fläche AREA, oberhalb dessen ein Capture vorliegt

6. Stimulationsvorrichtung nach einem der Ansprüche 1 bis 5, **dadurch gekennzeichnet, dass** die Auswerteeinheit ausgebildet ist, ein aufgenommenes elektrisches Signal laufend mit einem Grenzwert (zn) für die negative Signalamplitude zu vergleichen und im Falle des negativen Überschreitens des Grenzwertes (zn) ein einen Stimulationserfolg kennzeichnendes Signal abzugeben.

## Claims

1. A stimulation device, which has a stimulation unit, which is implemented to deliver electrical stimulation pulses to stimulate body tissue, as well as an analysis unit, which is implemented to record intracardial electrical signals in connection with the delivery of a stimulation pulse and analyze them to monitor a stimulation success,
wherein the analysis unit is implemented to detect those signal features in the intracardially recorded signal which are specific for a case of insufficient stimulation success and deliver a corresponding output signal,
wherein the analysis unit is implemented to chronologically assign a recorded electrical signal to a stimulation pulse and detect a feature of a polarization artifact as a signal feature in the recorded signal, and
to analyze the signal measured after passage of a blanking period after delivery of the stimulation pulse and, to detect a feature of a polarization artifact, to determine a first integral (INGR1) of the measured signal over the time, in which the signal measured after the blanking period runs above the signal amplitude during the blanking period.

2. The stimulation device according to Claim 1, **characterized in that** the analysis unit is implemented to determine a second integral (INGR2) of the measured signal over a time period which begins with the instant at which the first integral ends and which ends at the end of a predefined time window, which begins with the end of the blanking period.

3. The stimulation device according to Claim 1, in which the signal recorded after the delivery of a stimulation pulse is recorded in the form of chronologically discrete sampled values, **characterized in that** the analysis unit comprises a counter which is implemented to determine the number (CNT1) of the sampled values of the recorded signal which fall in the time over which the first integral is calculated.

4. The stimulation device according to Claim 2, **characterized in that** the analysis unit is implemented to produce an indicator flag (CROSS), whose value is a function of whether the measured signal crosses the signal amplitude existing during the blanking period during the time period for determining the second integral.

5. The stimulation device according to Claims 1 through 4, **characterized in that** the analysis unit is implemented to apply the following algorithm to an input signal:
If NEG_AMP < zn Then Capture
If CNT1 > w1 Then AREA = INGR1 + INGR2 Else AREA = INGR2
If AREA < a1 Then Non-Capture
Elseif CNT1 > w1 Then (If MAX_POS < zp Then Non-Capture Else Capture)
Elseif AREA > a2 Then Capture
Elseif CROSS = 1 Then Capture
Else Non-Capture
in which
NEG_AMP negative sampled values of the measured signal
INGR1 first integral of the measured signal over the time in which the signal measured during the blanking period runs above the signal amplitude during the blanking period.
INGR2 to determine second integral of the measured signal over a time period which begins with the instant at which the first integral ends, and which ends with the end of a predefined time window, which begins with the end of the blanking period
CNT1 number of sampled values, over which the first integral is calculated
AREA area which is formed by the first and the second integrals (INGR1, INGR2) as an identifier for a non-capture
MAX_POS maximum positive sampled value
CROSS indicator flag, whose value is a function of whether, during the time period for determining the second integral, the measured signal crosses the signal amplitude existing during the blanking period
w1 limiting value for a first predefined positive signal section, which is given by the number of sampled values over which the first integral is calculated (CNT1)
zn negative limiting value for the sampled values in relation to the signal amplitude during the blanking period
zp limiting value for the maximal positive sampled value MAX_POS if the positive signal section is wider than predefined by w1
a1 limiting value for an area AREA, which is calculated from the first and second integrals (INGR1, INGR2), as an identification for a non-capture
a2 limiting value for the area AREA, above which a capture exists.

6. The stimulation device according to one of Claims 1 through 5, **characterized in that** the analysis unit is implemented to compare a recorded electrical signal continuously to a limiting value (zn) for the negative signal amplitude and, in case of negatively exceeding the limiting value (zn), to deliver a signal identifying a stimulation success.

## Revendications

1. Dispositif de stimulation, qui présente une unité de stimulation, laquelle est réalisée pour délivrer des impulsions de stimulation électrique pour la stimulation de tissu corporel, ainsi qu'une unité d'analyse qui est conçue pour enregistrer des signaux électriques intracardiaques en rapport avec la délivrance d'une impulsion de stimulation et les analyser pour le contrôle d'un résultat de stimulation, l'unité d'analyse étant conçue pour détecter dans le signal enregistré au plan intracardiaque des attributs de signaux qui sont spécifiques pour un cas d'absence de résultat de stimulation et pour délivrer un signal sorti approprié, l'unité d'analyse étant conçue pour attribuer un signal électrique enregistré dans le temps à une impulsion de stimulation et pour détecter un attribut d'un artefact de polarisation comme attribut de signal dans le signal enregistré et pour analyser le signal mesuré après l'écoulement d'une période de suppression après la délivrance d'une impulsion de stimulation et pour déterminer, pour la détection d'un attribut d'un artefact de polarisation, une première intégrale (INGR1) du signal mesuré en fonction du temps, pendant lequel le signal mesuré après la période de suppression se situe au-dessus de l'amplitude de signal pendant l'arrêt de suppression.

2. Dispositif de stimulation selon la revendication 1, **caractérisé en ce que** l'unité d'analyse est conçue pour déterminer une seconde intégrale (INGR2) du signal mesuré pendant une période qui commence avec le moment pour lequel la première intégrale se termine, et qui se termine avec la fin d'une fenêtre de temps prédéfinie, laquelle commence à la fin de la période de suppression.

3. Dispositif de stimulation selon la revendication 1, dans lequel le signal enregistré après la délivrance d'une impulsion de stimulation est enregistré sous la forme de valeurs de balayage discrètes dans le temps, **caractérisé en ce que** l'unité d'analyse comprend un compteur qui est conçu pour déterminer le nombre (CNT1) des valeurs de balayage du signal enregistré qui tombent dans la période pendant laquelle la première intégrale est formée.

4. Dispositif de stimulation selon la revendication 2, **caractérisé en ce que** l'unité d'analyse est conçue pour former un drapeau indicateur (CROSS), dont la valeur dépend du fait que le signal mesuré croise ou non l'amplitude de signal régnant pendant la période de suppression pendant la durée pour la détermination de la seconde intégrale.

5. Dispositif de stimulation selon les revendications 1 à 4, **caractérisé en ce que** l'unité d'analyse est conçue pour appliquer l'algorithme suivant à un signal d'entrée :
If NEG_AMP < zn Then Capture
If CNT1 > w1 Then AREA = INGR1 + INGR2 Else AREA = INGR2
If AREA > a1 Then Non-Capture
Elseif CNT1 > w1 Then (If MAX_POS<zp Then Non-Capture Else Capture)
Elseif AREA > a2 Then Capture
Elseif CROSS = 1 Then Capture
Else Non-Capture
avec
NEG_AMP valeurs de balayage négatives du signal mesuré
INGR1 première intégrale du signal mesuré en fonction du temps pendant lequel le signal mesuré après la période de suppression est situé au-dessus de l'amplitude de signal pendant la période de suppression
INGR2 seconde intégrale du signal mesuré à déterminer en fonction d'une durée qui commence au moment auquel la première intégrale se termine, et qui commence à la fin d'une fenêtre de temps prédéfinie qui démarre avec la fin de la période de suppression
CNT1 Nombre de valeurs de balayage par lesquelles la première intégrale est formée
AREA Surface qui est formé de la première et de la seconde intégrales (INGR1, INGR2) comme attribut pour une non-capture
MAX_POS valeur de balayage positive maximale
CROSS drapeau indicateur, dont la valeur dépend du fait que le signal mesuré dépasse ou non l'amplitude de signal régnant pendant la période de suppression pendant la durée de la détermination de la seconde intégrale
w1 valeur limite pour une première partie de signal positive, qui est définie par le nombre des valeurs de balayage par lesquelles la première intégrale est formée (CNT1)
zn valeur limite négative pour les valeurs de balayage rapportées à l'amplitude de signal pendant la période de suppression
zp valeur limite pour la valeur de balayage positive maximale MAX_POS lorsque la partie de signal positive est plus large que la valeur prédéfinie par w1
a1 valeur limite pour une surface AREA qui est formée de la première et de la seconde intégrales (INGR1, INGR2) comme attribut pour une non-capture
a2 valeur limite pour la surface AREA, au-dessus de laquelle on a une capture

6. Dispositif de stimulation selon l'une quelconque des revendications 1 à 5, **caractérisé en ce que** l'unité d'analyse est formée pour comparer un signal électrique enregistré de façon continue avec une valeur limite (zn) pour l'amplitude de signal négative et pour délivrer un signal caractérisant un résultat de stimulation dans le cas du dépassement négatif de la valeur limite (zn).
